# EUROPEAN PATENT APPLICATION

(11) **EP 1 319 373 A1**
(43) Date of publication of application: **18.06.2003**
(21) Application number: 02258497.3
(22) Date of filing: 10.12.2002
(51) Int. Cl.: A61B 18/12, A61B 18/14

(54) **Electrosurgical apparatus and method for control of the same**

(30) Priority: 13.12.2001 GB 0129940
(71) Applicant: Nuvotek Ltd., Leeds LS12 2ND (GB)
(72) Inventor: Cobb, Gary, Long Eaton, Nottingham NG10 3RJ (GB)
(74) Representative: Wood, Graham

(57) **Abstract**

The invention relates to a surgical tool with an electricity supply provided from an electrosurgical generator. The apparatus includes an active or feed electrode and at least one return electrode, with the electrical supply controlled in order to allow and control the operations of the electrodes at any instant to operate in the predetermined active or return modes. This control is achieved by monitoring the dynamic feedback from the tool and altering the electrical output to maintain the active and return electrode conditions.

## Description

The invention to which this application relates is to a surgical tool and a system for the control thereof. The term "surgical tool" is used in this application to refer to a tool body to which one or a number of operating heads can be selectively attached. The ability to attach different format operating heads means that the tool can be used for a number of different surgical operations, as required.

Conventionally, there are two forms of electrosurgical apparatus, typically known as monopolar and bipolar. The monopolar system basically comprises an active electrode located in a tool which can be held by the surgeon and to which an electricity supply is provided which allows the same to be activated. A return electrode is provided in the form of a mat or plate on which the patient lies and which completes the circuit. A significant portion of the mat or plate is required to be retained in contact with the patient to ensure that the mat or plate does not start to act as the active electrode, as if it does start to act as an active electrode then it can cause severe burn injury to the patient.

The alternative form, known as bipolar, comprises the provision of the active and return electrodes within the same tool held by the surgeon and there is no need for a mat or plate to be provided.

Conventionally where the two electrodes are used in the tool, each is at a different electric potential with respect to the other and this is influenced by the changing impedance network, amplitude, frequency and phase variations of the underlying body tissue. In this situation, oscillation of RF energy can exist wherein RF current would flow from the return electrode until the potentials of the network have changed sufficiently for the oscillation reversal. These oscillations can occur at the RF base frequency and/or at harmonics of the same, and the coupling can therefore have adverse effects on the control of the RF energy to the body tissue.

In the operation of the tool in the bipolar system, it is important to ensure that one of the electrodes is always retained as the active electrode so that the tool can be controlled during the operation. In one type of bipolar both limbs of the tool are provided such that the current alternates between the two but this is only of practical use for coagulation procedures due to the alternation between electrodes. In the second type of bipolar there is provided a first electrode acting as the active electrode and a larger electrode such as the shaft of the tool, which size is required to be sufficiently large to ensure that the same acts as the return electrode. There is still a need for the electrical circuit which allows current to pass through the active electrode and return through the return electrode to be completed and then maintained and so in order for this form of tool to operate, the operation takes place with the active and return electrodes placed in a conductive fluid at the point of surgery at the body tissue. The requirement to use the conductive fluid is inconvenient, can be messy and generally complicates the operating procedure.

The aim of the present invention is to provide an electrosurgical tool and a control system therefore which includes an active and a return electrode and which allows control and differentiation between the two electrodes and hence the use of the tool for a range of surgical operations.

In a first aspect of the invention there is provided electrosurgical apparatus including a tool with a connection to an electrosurgical generator for the supply of an electricity supply to the tool, and controlling the same, said tool having at least first and second electrodes and characterised in that one of the electrodes is identified as an active electrode and the other is identified and maintained as the return electrode such that, in use, the tips of the active and return electrodes both contact the body tissue of the patient on which surgery is being performed.

Typically, the generator identifies and supplies the current to the electrosurgical tool in a manner to maintain the identified active and return electrodes in that condition during use thereby preventing switching between the active and return operation formats of the said electrodes.

In a further aspect of the invention there is provided a control system for an electrosurgical tool, said control system incorporating an electrosurgical generator for the generation, supply and control of electricity to the tool connected to the generator, said tool including at least first and second electrodes, said tips of the electrodes contactable with the body tissue in use and wherein the generator identifies one of the said electrodes as the active electrode and the other as the return electrode and then controls the supply of electricity to the tool to maintain the identified active and return electrodes in that condition during the operation.

Typically, the nomination and identification of the electrodes as active and return electrodes occurs during the factory setting of the electrosurgical generator.

In one embodiment, the nomination and identification of the active and return electrode is constant after the factory setting but in an alternative embodiment, and to allow specific surgical operations to be performed, the nomination and identification of the return and active electrodes can be changed over time and switched between said at least two electrodes in said tool. In this embodiment, it is envisaged that more than two electrodes can be provided in the tool with, at any one time, at least one of said electrodes being nominated as an active electrode and at least one of the remaining electrodes being nominated as the return electrode.

Typically the generator maintains the active and return electrodes in their operating conditions with respect to the impedance measurements and feedback received from the active and return electrodes.

In one preferred embodiment of the invention the system and tool can be used in a bipolar format whereby the return and active electrodes are both provided in the tool connected to the electrosurgical generator.

In a further aspect of the invention there is provided a method for the control of electrosurgical apparatus including an electrosurgical generator connected to a first electrode maintained in a body housing to be held by the user and a second electrode provided as a surface with which the patient contacts, said first electrode identified as the active electrode and, in use, the electricity supply to the said first electrode is controlled by the electrosurgical generator to ensure that the first electrode is maintained as the active electrode.

In this embodiment the tool and system are used in a monopolar format whereupon the active electrode is provided in the holder for the surgeon and connected to the electrosurgical generator and the return electrode is the plate or mat on which the patient is required to contact and said plate or mat is also connected to the electrosurgical generator, to complete the electrical circuit.

When used in the monopolar format in accordance with the invention, there is a distinct advantage over the prior monopolar systems in that the conventional monitoring mechanisms typically required to ensure that a sufficient portion of the plate or mat is in contact with the patient, is not required as the electrosurgical generator ensures that the active electrode is always maintained during operation and therefore there is no possibility for the plate or mat to become the active electrode due to the lack of contact with the patient.

In accordance with a further aspect of the invention there is provided a method for controlling the electricity supply to an electrosurgical tool having at least two electrodes and controlling the operation of the same such that one acts as an active electrode and the other as a return electrode in a controlled manner, said electricity supply controlled by an electrosurgical generator connected to the tool and characterised in that the generator controls the RF output from the generator to the tips of the electrodes to oscillate between the electrodes, dependant on the contact geometry of the tips at the body tissue.

In one embodiment the RF oscillation is controlled by the generator with reference to the dynamic feedback of real time measurement of an impedance network to control the continuous RF output at the nominated active electrode within the plurality of electrodes at each instant. Typically the impedance feedback is monitored which varies with changing resistive and reactive parts with the reactive part comprising changing capacitance and inductance values.

There is thus provided an electrosurgical tool for use with the invention, said tool including a connection to an electrosurgical generator for supplying an electricity supply, and controlling the same, to the tool and at least first and second electrodes and wherein one of the electrodes is identified and maintained as the active electrode and the other is identified and maintained as the return electrode such that, in use, the tips of the active and return electrodes can both safely contact the body tissue of the patient without the need for a conductive fluid to be provided at the area of surgery..

Specific embodiments of the invention will now be described with reference to the accompanying drawings, wherein:-
Figure 1 illustrates a bipolar format of the invention;
Figure 2 illustrates the tip of a tool in cross section for use in the format of Figure 1; and
Figure 3 illustrates a monopolar format of the invention.

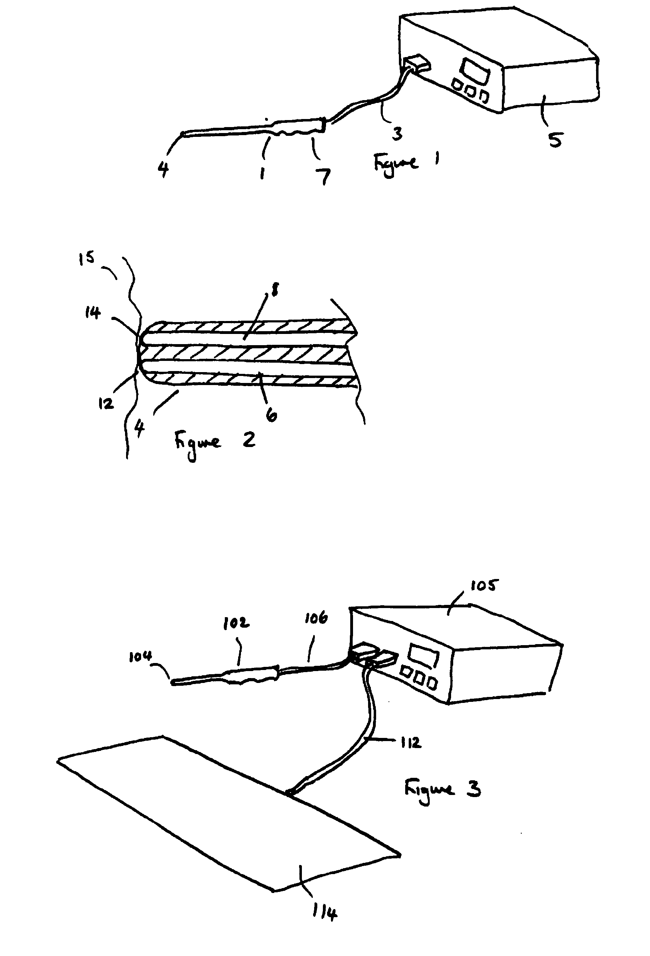

Figures 1 and 2 illustrate one format of the tool and system in accordance with the invention wherein the tool 1 includes a connection 3 to an electrosurgical generator 5 which will be described in more detail below. The tool has an operating end 4 a handle 7 and, between the connection 3 to the electrosurgical generator and the operating end 4, at least two electrodes, active electrode 6, and return electrode 8 which run along the length of the tool 1. The tips 12 and 14 of the electrodes 6, 8 respectively, lie in substantially the same plane at the operating end 4 of the tool. This means that when the tool 1 is in use in relation to patient body tissue 15, both the tips 12, 14 of the electrodes 6, 8 are in contact with the tissue.

In the bipolar format of Figures 1 and 2 the body tissue 15 between the tip of the electrode 6 and the tip of the electrode 8 completes the electrical circuit including the generator and the electrodes and the connection 3 between the same.

There is now described a method of controlling the output to the electrodes of the format of Figures 1 and 2 wherein the two electrodes 6, 8, or potentially a greater number of electrodes, are in contact with the tissue target site. The control mechanism builds on the system described in the applicants granted patent, as follows.

When an RF (radio frequency) circuit is activated between the electrosurgical generator 5, and the identified active electrode 6, body tissue 15 and identified return electrode 8, the electrical potential of the electrode tips will oscillate at a given frequency. In accordance with the invention the generator controls the electricity supply to ensure that the active electrode 6 and return electrode 8 remain in those operating conditions. As this potential is normally continually variable, the RF output from the generator is controlled to oscillate between the electrodes, dependant on the contact geometry of the tips at the target site such as bipolar forceps or similar configuration. As the RF potential gradient of each electrode 6, 8 varies, charged particles in the tissue under the electrodes are established. This is proportional to the potential of the RF electrical gradient and in turn creates a net charge so that a current will flow from one electrode 6, through the body tissue and to the other electrode 8 which will be at a lower RF potential. As there is variable impedance, said impedance being a complex component having a changing resistive and reactive part, then the reactive portion is an ever-changing network of capacitance and inductance. The reactance of the tissue under the influence of the RF potential creates an oscillating capacitive as well as an inductive network. This would allow, if not controlled in accordance with the invention, the RF potential from one electrode to move to the other. However, due to the invention which uses a method of tuning and measuring impedance feedback, it is possible to control the oscillation of the RF potential dynamically across the electrodes 6, 8 as the same are in close geometric proximity to each other, so that the identified active electrode is retained as the active electrode.

This invention overcomes many problems conventionally associated with RF delivery using two or more electrodes which are in direct contact with the tissue target area. The application of the RF energy from the electrode geometry, which is in close proximity to the tissue target site, has many advantages, not least of which is that it offers greater localisation and control of the RF energy delivery since the active electrode tip is almost a point source. However, in order to achieve accurate RF delivery, considerable consideration and monitoring must be given to the tissue effects, as described in the applicant's granted patent and which are included herein by reference, where recognition is reflected to take into account for RF energy dissipation and storage within the tissues. In this invention the method is extended to control the RF energy to a specific electrode which then becomes independent of the variables described above and also the geometry and proximity of the active and return electrodes becomes less significant.

Thus, devices which employ multiple electrodes are critically and beneficially affected by this invention as until now it has not been possible to affect device geometry in a way to allow accurate controlled RF delivery to the body tissue with multiple electrodes in contact with the tissue where there is an active and return electrode.

This invention therefore provides a method of controlling the RF oscillation from multiple electrodes using dynamic feedback of real time measurement of an impedance network and hence control the continuous RF energy from the nominated and identified active electrode within the array of electrodes. The feature can be selected via software algorithms as a default fixed and/or, alternatively a rotational or changing feature to suit the surgical requirements.

Figure 3 illustrates another format of the invention in use wherein there is provided a tool 102 which has an operating end 104 and a connection 106 to an electrosurgical generator 105. Running along the length of the tool 102 between the connection to the generator and the operating end is an active electrode plate or mat 114 is provided which acts as the return electrode via connection 112 to the electrosurgical generator 105. This is known as the monopolar format in which the patient body when lying on the plate 114 completes the circuit, when the tool operating end 104 contacts the body tissue

In practise with the invention, in whichever format the first part of the system involves localising and fixing the RF energy to a single nominated and identified active electrode within the available electrodes and also nominating and identifying the return electrode, if there are a number of electrodes remaining.

Signals and information which emanate from the nominated and identified active and return electrodes are then acquired and processed, via the electrosurgical generator to determine the location of the single or multiple active electrodes and the relative location of the return electrode, and hence maintain the same in that format.

## Claims

1. Electrosurgical apparatus including a tool with a connection to an electrosurgical generator for the supply of an electricity supply to the tool, and controlling the same, said tool having at least first and second electrodes and **characterised in that** one of the electrodes is identified as an active electrode and the other is identified and maintained as the return electrode such that, in use, the tips of the active and return electrodes both contact the body tissue of the patient on which surgery is being performed.

2. Electrosurgical apparatus according to claim 1 wherein the electrosurgical generator identifies and supplies current to the electrosurgical tool to maintain the identified active and return electrodes in that condition during use of the tool.

3. A control system for an electrosurgical tool, said control system incorporating an electrosurgical generator for the generation, supply and control of electricity to the tool connected to the generator, said tool including at least first and second electrodes, said tips of the electrodes contactable with body tissue in use and **characterised in that** the generator identifies one of the said electrodes as the active electrode and the other as the return electrode and then controls a supply of electricity to the tool to maintain the identified active and return electrodes **in that** condition during use of the tool.

4. A system according to claim 3 wherein the nomination and identification of the electrodes of the tool as active and return occurs during the factory setting of the electrosurgical generator connected to the tool.

5. A system according to claim 4 **characterised in that** once nominated, the active and return electrodes are constantly used in the same configuration.

6. A system according to claim 3 **characterised in that** the nomination and identification of the return and active electrodes of the tool change over time by switching.

7. A system according to claim 3 **characterised in that** more than two electrodes are provided in the tool with, at any one time in use, at least one of said electrodes being nominated as an active electrode and at least one of the remaining electrodes nominated as a return electrode.

8. A system according to claim 3 **characterised in that** the generator maintains the active and return electrodes in their operating conditions with respect to the impedance measurement and feedback received from the tool in use.

9. A method for the control of electrosurgical apparatus including an electrosurgical generator connected to a first electrode maintained in a body housing to be held by the user and a second electrode provided as a surface with which the patient contacts, said first electrode identified as the active electrode and, in use, the electricity supply to the said first electrode is controlled by the electrosurgical generator to ensure that the first electrode is maintained as the active electrode.

10. A method according to claim 9, **characterised in that** the active electrode is provided in a housing for the surgeon and connected to the electrosurgical generator and the return electrode surface is a plate connected to the electrosurgical generator to complete the electrical circuit.

11. A method according to claim 10 **characterised in that** the electrosurgical generator maintains the electrode in the tool as the active electrode throughout the use of the apparatus.

12. A method for controlling the electricity supply to an electrosurgical tool having at least two electrodes and controlling the operation of the same such that one acts as an active electrode and the other as a return electrode in a controlled manner, said electricity supply controlled by an electrosurgical generator connected to the tool and **characterised in that** the generator controls the RF output from the generator to the tips of the electrodes to oscillate between the electrodes, dependant on the contact geometry of the tips at the body tissue.

13. A method according to claim 12 **characterised in that** the RF oscillation is controlled by the generator with reference to the dynamic feedback of real time measurement of an impedance network to control the continuous RF output at the nominated active electrode within the plurality of electrodes at each instant.

14. A method according to claim 13 **characterised in that** the impedance feedback is monitored which varies with changing resistive and reactive parts with the reactive part comprising changing capacitance and inductance values.
